(19) Europäisches Patentamt
European Patent Office
Office européen des brevets

(11) **EP 4 249 907 A1**

(12) **EUROPEAN PATENT APPLICATION**
published in accordance with Art. 153(4) EPC

(43) Date of publication:
**27.09.2023 Bulletin 2023/39**

(21) Application number: **20961511.1**

(22) Date of filing: **10.11.2020**

(51) International Patent Classification (IPC):
***G01N 33/00*** *(2006.01)*

(52) Cooperative Patent Classification (CPC):
**G01N 33/00**

(86) International application number:
**PCT/JP2020/041928**

(87) International publication number:
**WO 2022/101983 (19.05.2022 Gazette 2022/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Perkinelmer Japan G.K.**
**Yokohama-shi, Kanagawa, 240-0005 (JP)**

(72) Inventors:
• SUZUKI Toshiyuki
**Yokohama-shi, Kanagawa 240-0005 (JP)**
• FURUKAWA Makoto
**Yokohama-shi, Kanagawa 240-0005 (JP)**

(74) Representative: **Richly & Ritschel Patentanwälte PartG mbB**
**Sattlerweg 20**
**51429 Bergisch Gladbach (DE)**

(54) **SAMPLE CLASSIFICATION METHOD**

(57)     A method of classifying a sample of material includes a first analysis step of obtaining a first set of analysis values using a first analysis method at time 11, a second analysis step of obtaining a second set of analysis values using the first analysis method at time t2 which is later than the time 11, a first set of arc-tangents calculating step of obtaining a set of set of arc-tangents $\theta_{1n}$ of respective ratios of analysis value differences to a time difference (t2 - t1), a first principal component analysis step of performing a principal component analysis of the set of set of arc-tangents $\theta_{1n}$ to obtain a principal component score comp. 1, a third analysis step of obtaining a third set of analysis values at time t3 using a second analysis method different from the first analysis method, a fourth analysis step of obtaining a fourth set of analysis values using the second analysis method at time t4 which is later than the time t3, a second set of arc-tangents calculation step of obtaining a set of set of arc-tangents $\theta_{2n}$ of respective ratios of analysis value differences to a time difference (t4 - t3), a second principal component analysis step of performing principal component analysis on the set of set of arc-tangents $\theta_{2n}$ to obtain a principal component score comp.2, and a classification step of classifying the sample of material based on the principal component score comp.1 and the principal component score comp.2.

FIG. 1

**Description**

[Technical Field]

The present invention relates to a method of classifying a sample of material.

[Background Art]

In the course of data analysis, samples of material with similar characteristics may sometimes be classified into the same set based on analysis data. Correlations between multiple pieces of analysis data may also sometimes be evaluated. Appropriate transformation or processing of analysis data is important for appropriate classification and correlation evaluation.

Patent Document 1 discloses a method including the step of calculating degradation rates and apparent synthesis rates of transcription products of genes and then calculating net synthesis rates of the transcription products of genes from the apparent synthesis rates and the degradation rates; the step of clustering wave patterns of the net synthesis rates; and the step of, based on the clustering, classifying genes belonging to the same cluster as genes having at least one same characteristic.

Patent Document 2 discloses a signal processing device that uses rotation matrices $Qa$ and $Q\alpha$ indicating sinusoidal functions, includes an input that receives a sample $x(n+1)$ at time $n+1$, another input that receives a reference sample $y(n+1)$ at the same time, and a calculation means that performs an operation in response to a processing device that minimizes a signal $e(n+1)$ representing the difference between the received signal and the reference sample, and performs a least squares method, wherein the calculation means includes a normalization means that normalizes the input sample with respect to their energy function.

Patent Document 3 discloses an image processing method including the step of storing a mapping transform at a fourth computer memory location and the step of acquiring, for each image pixel, image pixel data including data elements of three color components of each image pixel, and transforming the color component data elements of each image pixel using the mapping transform to calculate normalized image pixel data for each image pixel.

Patent Document 4 discloses a method that preprocesses image data by decomposing the image data into overlapping sub-images, applying PCA to the sub-images to derive a first principal component image, thresholding the first principal component image to produce a binary image of blobs and background, rejecting blobs adjacent to or intersecting sub-image boundaries, filling holes in blobs, rejecting blobs too small to correspond to potential mitotic figures, and reassembling the sub-images into a single image for image region profile measurement.

[Citation List]

[Patent Document]

[Patent Document 1]
PCT International Publication No. WO2008-102825
[Patent Document 2]
Japanese Unexamined Patent Application, First Publication No. H5-324272
[Patent Document 3]
Published Japanese Translation No. 2018-535471 of the PCT International Publication
[Patent Document 4]
Japanese Unexamined Patent Application, First Publication No. 2008-77677

[Summary of Invention]

[Technical Problem]

However, since pieces of analysis data obtained by different analysis methods have different intensity scales, it was difficult to compare pieces of analysis data having different intensity scales with the data processing methods described in Patent Documents 1 to 5. As a result, there was a problem that it was difficult to classify a sample of material using the correlation of analysis data having different intensity scales.

On the other hand, a method that performs principal component analysis on analysis data having different intensity scales has been reported. However, when analysis data for principal component analysis is time series data having static information which is a physical quantity and dynamic information which is time, it is difficult to perform principal component analysis on different time series data using a uniform model. Therefore, it was difficult to compare different time series data even using principal component analysis.

A method that analyzes time series data by combining time series modeling that performs discrete Fourier transform, a dynamic factor model, or ANOVA with principal component analysis has also been reported. However, it was difficult to simultaneously express time and physical quantities obtained from different analysis methods as properties of the sample of material. It was also difficult to compare different time series data even when the above methods were combined with principal component analysis.

Thus, with the methods of the related art, there was a problem that it was difficult to classify a sample of material based on different time series data obtained by different analysis methods and their relationships.

The present invention has been made to solve the above problems and it is an object of the present invention to provide a method of classifying a sample of material that enables comparison of pieces of time series data obtained by different analysis methods.

[Solution to Problem]

To solve the above problems, the present invention provides the following means.

(1) An aspect of a method of classifying a sample of material according to the present invention includes a first analysis step of analyzing a sample of material at time t1 using a first analysis method to obtain a first set of analysis values ($I_{111}$, $I_{112}$...$I_{11n}$), a second analysis step of analyzing the sample of material using the first analysis method at time t2 which is later than the time t1 to obtain a second set of analysis values ($I_{121}$, $I_{122}$, ..., $I_{12n}$), a first set of arc-tangents calculating step of obtaining an set of arc-tangents $\theta_{1n}$ of a ratio of an analysis value difference ($I_{12n}$ - $I_{11n}$) to a time difference (t2 - t1) for each pair of analysis values of the first and second sets of analysis values, a first principal component analysis step of performing principal component analysis on a plurality of the set of arc-tangents $\theta_{1n}$ to obtain a principal component score comp. 1, a third analysis step of analyzing the sample of material at time t3 using a second analysis method different from the first analysis method to obtain a third set of analysis values ($I_{231}$, $I_{232}$, ..., $I_{23n}$), a fourth analysis step of analyzing the sample of material using the second analysis method at time t4 which is later than the time t3 to obtain a fourth set of analysis values ($I_{241}$, $I_{242}$, ..., $I_{24n}$), a second set of arc-tangents calculation step of obtaining an set of arc-tangents $\theta_{2n}$ of a ratio of an analysis value difference ($I_{24n}$ - $I_{23n}$) to a time difference (t4 - t3) for each pair of analysis values of the third and fourth sets of analysis values, a second principal component analysis step of performing principal component analysis on a plurality of the set of arc-tangents $\theta_{2n}$ to obtain a principal component score comp.2, and a classification step of plotting the principal component score comp.1 on a first axis and the principal component score comp.2 on a second axis in a coordinate system having the first and second axes and classifying the sample of material based on the principal component scores of the second analysis method with respect to the principal component scores of the first analysis method.

(2) The method according to (1), further including a fifth analysis step of analyzing the sample of material using the second analysis method at time t5 which is later than the time t4 to obtain a fifth set of analysis values ($I_{251}$, $I_{252}$, ..., $I_{25n}$), a third set of arc-tangents calculation step of obtaining an set of arc-tangents $\theta_{21n}$ of a ratio of a value difference ($I_{25n}$ - $I_{24n}$) to a time difference (t5 - t4) for each pair of analysis values of the fourth and fifth sets of analysis values, and a third principal component analysis step of performing principal component analysis on the set of arc-tangents $\theta_{21n}$ to obtain a principal component score comp.3, wherein the classification step includes plotting the principal component score comp.1 on the first axis and the principal component score comp.3 on the second axis in the coordinate system having the first and second axes to classify the sample of material.

(3) The method according to (2), wherein the classification step includes comparing a first coordinate position represented by the principal component score comp.1 and the principal component score comp.2 and a second coordinate position represented by the principal component score comp.1 and the principal component score comp.3 which are plotted in the coordinate system.

(4) The method according to (3), wherein the classification step includes comparing the first coordinate position and the second coordinate position based on a direction of displacement from the first coordinate position to the second coordinate position.

(5) The method according to (2), further including a sixth analysis step of analyzing the sample of material at time t6 using a third analysis method different from the first and second analysis methods to obtain a sixth set of analysis values ($I_{361}$, $I_{362}$, ..., $I_{36n}$), a seventh analysis step of analyzing the sample of material using the third analysis method at time t7 which is later than the time t6 to obtain a seventh set of analysis values ($I_{371}$, $I_{372}$, ..., $I_{37n}$), a fourth set of arc-tangents calculation step of obtaining an set of arc-tangents $\theta_{3n}$ of a ratio of an analysis value difference ($I_{37n}$ - $I_{36n}$) to a time difference (t7 - t6) for each pair of analysis values of the sixth and seventh sets of analysis values, and a fourth principal component analysis step of performing principal component analysis on the set of arc-tangents

$\theta_{3n}$ to obtain a principal component score comp.4, wherein the classification step includes plotting the principal component score comp.4 on a third axis, the principal component score comp.1 on the first axis, and the principal component score comp.2 on the second axis in the coordinate system having the first, second, and third axes.

(6) The method according to (5), further including an eighth analysis step of analyzing the sample of material using the third analysis method at time t8 which is later than the time t7 to obtain an eighth set of analysis values ($I_{381}$, $I_{382}$, ..., $I_{38n}$), a fifth set of arc-tangents calculation step of obtaining an set of arc-tangents $\theta_{31n}$ of a ratio of an analysis value difference ($I_{38n} - I_{37n}$) to a time difference (t8 - t7) for each pair of analysis values of the seventh and eighth sets of analysis values, and a fifth principal component analysis step of performing principal component analysis on the set of arc-tangents $\theta_{31n}$ to obtain a principal component score comp.5, wherein the classification step includes plotting the principal component score comp.5 on the third axis, the principal component score comp.1 on the first axis, and the principal component score comp.3 on the second axis in the coordinate system having the first, second, and third axes.

(7) The method according to (6), wherein the classification step includes comparing a third coordinate position represented by the principal component score comp.1, the principal component score comp.2, and the principal component score comp.4 and a fourth coordinate position represented by the principal component score comp.1, the principal component score comp.3, and the principal component score comp.5 which are plotted in the coordinate system.

(8) The method according to (7), wherein the classification step includes comparing the third coordinate position and the fourth coordinate position based on a direction of displacement from the third coordinate position to the fourth coordinate position.

(9) The method according to (1), wherein the first and second analysis methods are methods for analyzing different physical quantities.

[Advantageous Effects of Invention]

According to the above aspects of the present invention, it is possible to provide a method of classifying a sample of material that enables comparison of pieces of data having different intensity scales obtained by different analysis methods.

[Brief Description of Drawings]

FIG. 1 is a conceptual diagram when data is normalized in an embodiment of the present invention.
FIG. 2 is a diagram showing the rate of mass loss due to drying based on TG data.
FIG. 3 is a dendrogram based on $\theta$ values obtained by IR.
FIG. 4 is a dendrogram based on $\theta$ values obtained by ICP-MS.
FIG. 5 is a dendrogram based on $\theta$ values obtained by GC/MS.
FIG. 6 is a dendrogram of combinations of $\theta$ values obtained by IR, ICP-MS, and GC/MS.
FIG. 7 is a diagram showing PCA results of data groups of a combination of IR, ICP-MS, and GC/MS.
FIG. 8 is a dendrogram of combinations of $\theta$ values obtained by IR and ICP-MS.
FIG. 9 is a diagram showing plots of principal component scores obtained from IR, ICP-MS, and GC/MS data.
FIG. 10 is a diagram showing the movement of principal component scores over time.
FIG. 11 is a diagram showing PCA loadings plots of data of FIG. 10.

[Description of Embodiments]

Embodiments

A method of classifying a sample of material according to an embodiment of the present invention will be described below.
The method of classifying a sample of material according to an embodiment of the present invention includes a first analysis step, a second analysis step, a first set of arc-tangents calculation step, a first principal component analysis step, a third analysis step, a fourth analysis step, a second set of arc-tangents calculation step, a second principal component analysis step, and a classification step.

First analysis step

In the first analysis step of the method of classifying a sample of material according to the embodiment of the present invention, a sample of material is analyzed at time t1 using a first analysis method. As a result, a first set of analysis values ($I_{111}$, $I_{112}$, ..., $I_{11n}$) is obtained. The first analysis method can be selected according to the sample of material to be analyzed. Here, n represents a positive integer.

Second analysis step

In the second analysis step of the method of classifying a sample of material according to the embodiment of the present invention, the sample of material is analyzed using the first analysis method at time t2 which is later than time t1 and as a result a second set of analysis values ($I_{121}$, $I_{122}$, ..., $I_{12n}$) is obtained.

First set of arc-tangents calculation step

In the first set of arc-tangents calculation step of the method of classifying a sample of material according to the embodiment of the present invention, ($I_{12n}$ - $I_{11n}$)/(t2 - t1) which is a ratio of an analysis value difference ($I_{12n}$ - $I_{11n}$) to a time difference (t2 - t1) is calculated for each pair of analysis values of the first and second sets of analysis values. Then, $\tan((I_{12n} - I_{11n})/(t2 - t1))^{-1}$ which is the set of arc-tangents $\theta_{1n}$ of ($I_{12n}$ - $I_{11n}$)/(t2 - t1) is obtained.

First principal component analysis step

In the first principal component analysis step of the method of classifying a sample of material according to the embodiment of the present invention, principal component analysis is performed on a plurality of set of arc-tangents $\theta_{1n}$ to obtain a principal component score comp.1 (a principal component score).

Third analysis step

In the third analysis step of the method of classifying a sample of material according to the embodiment of the present invention, the sample of material is analyzed at time t3 using a second analysis method different from the first analysis method. As a result, a third set of analysis values ($I_{231}$, $I_{232}$, ..., $I_{23n}$) is obtained. The fact that the first and second analysis methods differ means that the analysis methods differ in something as well as in the analyzed time. For example, a method of analyzing Cr by ICP-MS and a method of analyzing Mo by ICP-MS which is performed at a different time are different analysis methods and a method of analyzing Cr by ICP-MS and a method of analyzing Cr by EDS are also different analysis methods. Analysis values obtained by the first and second analysis methods may be those of the same physical quantity or different physical quantities. The sequential relationship between time t3 and times t1 and t2 is not particularly limited.

Fourth analysis step

In the fourth analysis step of the method of classifying a sample of material according to the embodiment of the present invention, the sample of material is analyzed using the second analysis method at time t4 which is later than time t3. As a result, a fourth set of analysis values ($1_{241}$, $I_{242}$, ..., $I_{24n}$) is obtained.

Second set of arc-tangents calculation step

In the second set of arc-tangents calculation step of the method of classifying a sample of material according to the embodiment of the present invention, ($I_{24n}$ - $I_{23n}$)/(t4 - t3) which is a ratio of an analysis value difference ($I_{24n}$ - $I_{23n}$) to a time difference (t4 - t3) is calculated for each pair of analysis values of the third and fourth sets of analysis values. Then, $\tan((I_{24n} - I_{23n})/(t4 - t3))^{-1}$ which is the set of arc-tangents $\theta_{2n}$ of ($I_{24n}$ - $I_{23n}$)/(t4 - t3) is obtained.

Second principal component analysis step

In the second principal component analysis step of the method of classifying a sample of material according to the embodiment of the present invention, principal component analysis is performed on a plurality of set of arc-tangents $\theta_{2n}$ to obtain a principal component score comp.2.

Classification step

In the classification step of the method of classifying a sample of material according to the embodiment of the present invention, the principal component score comp.1 and the principal component score comp.2 are plotted in a coordinate system having a first axis and a second axis. The principal component score comp. 1 and the principal component score comp.2 are plotted on different axes. For example, the principal component score comp. 1 may be plotted on the first axis and the principal component score comp.2 may be plotted on the second axis. Then, the sample of material is classified based on the plotted principal component scores of the first analysis method and the second analysis method. The coordinate system may have three or more axes. A principal component score plotted on an axis other than the first and second axes may be calculated from data obtained by the same analysis method as the first analysis method or the second analysis method or may be calculated from data obtained by a different analysis method.

Results obtained by different analytical devices are results expressed with different dimensions and dimensionalities and different intensities such as the number of measurable components and the magnitude and unit of the detected amount. It is difficult to perform, using a uniform model, principal component analysis (PCA) on groups of different types of data obtained by such analytical devices. Principal component scores (components) obtained by PCA are greatly affected by data with high intensities or large variances and therefore unless different types of data are analyzed with intensities that are treated comparably, the influence of data with high data intensities will be highly reflected in principal component scores and as a result the principal component scores that reflect the data with high data intensities will be extracted.

On the other hand, if the influence of data intensities can be normalized, it is possible to obtain the overall correlations of data using PCA and visualize them as mutual positional relationships. The present inventor has found this and completed the present invention.

According to the method of classifying a sample of material according to the embodiment of the present invention, pieces of data obtained by the first and second analysis methods are converted into normalized data groups incorporating time through the first and second set of arc-tangents calculation steps, respectively. Therefore, results of different physical quantities obtained from a group of devices can be normalized not only as simple comparisons of entries, amounts, or proportions but also as changes over time. As a result, the properties of the sample of material can be expressed simultaneously as static information, which is a physical quantity, and time.

Further, the first and second principal component analysis steps enable comparison of pieces of time series data obtained by different analysis methods. As a result, samples of material can be classified according to time series data and relationships between different time series data.

In addition, time series data having different intensity scales can be normalized by calculating the set of arc-tangents of the ratio of the analysis value difference to the time difference. This enables comparison of pieces of time series data having different intensity scales. Therefore, it is possible to classify the sample of material without individually analyzing each analysis method to determine the attributes of the sample of material. It is also possible to classify the sample of material without advanced knowledge of each analysis method. Thus, even a person or machine without advanced knowledge can easily classify the sample of material.

When analysis values used for classification contain data indicating a feature desired to be classified, the principal component score group is expressed as a cluster. On the other hand, when analysis values do not contain data indicating a feature desired to be classified, the principal component score group is expressed as a variance. Therefore, it is possible to determine whether a sufficient analysis method has been used to classify the sample of material with a feature desired to be classified. As a result, it is possible to determine whether the type and results of the analysis method used for analysis of properties are appropriate. Further, the results of determining whether the type and results of the analysis method are appropriate can be fed back to the selection of the analysis method. Thereby, the sample of material can be classified accurately. Furthermore, advanced knowledge of the analysis method is not required to determine whether the type and results of the analysis method are appropriate. As a result, even a person or machine without advanced knowledge can easily classify the sample of material.

Modifications

Next, a method of classifying a sample of material according to a modification of the embodiment of the present invention will be described. Descriptions of configurations similar to those of the above embodiment may sometimes be omitted.

The method of classifying a sample of material according to the embodiment of the present invention may include a fifth analysis step, a third set of arc-tangents calculation step, and a third principal component analysis step in addition to the configuration of the above embodiment.

Fifth analysis step

The fifth analysis step of the method of classifying a sample of material according to the embodiment of the present invention may analyze the sample of material using the second analysis method at time t5 which is later than time t4. Thereby, a fifth set of analysis values ($I_{251}$, $I_{252}$, ..., $I_{25n}$) is obtained.

Third set of arc-tangents calculation step

In the third set of arc-tangents calculation step of the method of classifying a sample of material according to the embodiment of the present invention, ($I_{25n}$ - $I_{24n}$)/(t5 - t4) which is a ratio of an analysis value difference ($I_{25n}$ - $I_{24n}$) to a time difference (t5 - t4) may be calculated for each pair of analysis values of the fourth and fifth sets of analysis values. Then, $\tan((I_{25n} - I_{24n})/(t5 - t4))^{-1}$ which is the set of arc-tangents $\theta_{21n}$, of ($I_{25n}$, - $I_{24n}$)/(t5 - t4) may be obtained.

Third principal component analysis step

In the third principal component analysis step of the method of classifying a sample of material according to the embodiment of the present invention, principal component analysis may be performed on the set of arc-tangents $\theta_{21n}$. to obtain a principal component score comp.3.

Classification step

When the method of classifying a sample of material according to the embodiment of the present invention further includes the fifth analysis step, the third set of arc-tangents calculation step, and the third principal component analysis step, the principal component score comp. 1 may be plotted on the first axis and the principal component score comp.3 may be plotted on the second axis in the coordinate system having the first and second axes in the classification step. The principal component score comp.2 may also be plotted on the second axis. The principal component score comp.3 and the principal component score comp.2 are results obtained by the same analysis method. Further, the principal component score comp.3 represents information on the sample of material later in time than the principal component score comp.2. Thus, the plotting method described above makes it easy to extract time-dependent properties of the sample of material. As a result, the sample of material can be classified more accurately using the time-dependent properties of the sample of material. The coordinate system in which the principal component score comp. 1 and the principal component score comp.2 are plotted and the coordinate system in which the principal component score comp.1 and the principal component score comp.3 are plotted may be the same or different.

A first coordinate position represented by the principal component score comp. 1 and the principal component score comp.2 and a second coordinate position represented by the principal component score comp.1 and the principal component score comp.3, which are plotted in the coordinate system, may be compared in the classification step. This makes changes in the properties of the sample of material over time more obvious. As a result, the sample of material can be classified more accurately. A method of comparing the first coordinate position and the second coordinate position may use, for example, the direction or magnitude of displacement from the first coordinate position to the second coordinate position. This makes it possible to more quantitatively extract time-dependent properties of the sample of material. As a result, the sample of material can be classified more accurately.

A method of classifying a sample of material according to a modification of the embodiment of the present invention may further include a sixth analysis step, a seventh analysis step, a fourth set of arc-tangents calculation step, and a fourth principal component analysis step.

Sixth analysis step

In the eighth analysis step of the method of classifying a sample of material according to the modification of the embodiment of the present invention, the sample of material may be analyzed at time t6 using a third analysis method different from the first and second analysis methods. Thereby, a sixth set of analysis values ($I_{361}$, $I_{362}$, ..., $I_{36n}$) is obtained. Here, n represents a positive integer.

Seventh analysis step

In the seventh analysis step of the method of classifying a sample of material according to the modification of the embodiment of the present invention, the sample of material may be analyzed using the third analysis method at time t7 which is later than time t6. Thereby, a seventh set of analysis values ($I_{371}$, $I_{372}$, ..., $I_{37n}$) is obtained.

Fourth set of arc-tangents calculation step

In the fourth set of arc-tangents calculation step of the method of classifying a sample of material according to the modification of the embodiment of the present invention, $(I_{37n} - I_{36n})/(t7 - t6)$ which is a ratio of an analysis value difference $(1_{37n}, - I_{36n})$ to a time difference $(t7 - t6)$ may be calculated for each pair of analysis values of the sixth and seventh sets of analysis values. Then, $\tan((I_{37n}, - I_{36n})/(t7 - t6))^{-1}$ which is the set of arc-tangents $\theta_{3n}$ of $(I_{37n}, - I_{36n})/(t7 - t6)$ may be obtained.

Fourth principal component analysis step

In the fourth principal component analysis step of the method of classifying a sample of material according to the modification of the embodiment of the present invention, principal component analysis may be performed on the set of arc-tangents $\theta_{3n}$ to obtain a principal component score comp.4.

Classification step

In the classification step of the method of classifying a sample of material according to another modification of the embodiment of the present invention when the sixth analysis step, the seventh analysis step, the fourth set of arc-tangents calculation step, and the fourth principal component analysis step are included, the principal component score comp.4 may be plotted on a third axis, the principal component score comp. 1 may be plotted on a first axis, and the principal component score comp.2 may be plotted on a second axis in a coordinate system having the first, second, and third axes. This enables comparison of pieces of time series data obtained by three different analysis methods. As a result, the sample of material can be classified more accurately.

The principal component score comp.3 may be plotted on the second axis. The principal component score comp.2 and the principal component score comp.3 are results obtained by the same analysis method. Further, the principal component score comp.3 represents information on the sample of material later in time than the principal component score comp.2. In addition, it is possible to compare pieces of time series data obtained by three different analysis methods. Thus, the plotting method described above makes it easy to extract time-dependent properties of the sample of material. As a result, the sample of material can be classified more accurately using the time-dependent properties of the sample of material. The coordinate system in which the principal component score comp.4, the principal component score comp.1, and the principal component score comp.2 are plotted and the coordinate system in which the principal component score comp.4, the principal component score comp.1, and the principal component score comp.3 are plotted may be the same or different.

A method of classifying a sample of material according to a modification of the embodiment of the present invention may further include an eighth analysis step, a fifth set of arc-tangents calculation step, and a fifth principal component analysis step.

Eighth analysis step

In the eighth analysis step of the method of classifying a sample of material according to the modification of the embodiment of the present invention, the sample of material may be analyzed using the third analysis method at time t8 which is later than time t7. Thereby, an eighth set of analysis values $(I_{381}, I_{382}, ..., I_{38n\cdot})$ is obtained.

Fifth set of arc-tangents calculation step

In the fifth set of arc-tangents calculation step of the method of classifying a sample of material according to the modification of the embodiment of the present invention, $(I_{38n\cdot} - I_{37n})/(t8 - t7)$ which is a ratio of an analysis value difference $(I_{38n} - I_{37n})$ to a time difference $(t8 - t7)$ may be calculated for each pair of analysis values of the seventh and eighth sets of analysis values. Then, $\tan((I_{38n} - I_{37n})/(t8 - t7))^{-1}$ which is the set of arc-tangents $\theta_{31n}$, of $(I_{38n}, - I_{37n})/(t8 - t7)$ may be obtained.

Fifth principal component analysis step

In the fifth principal component analysis step of the method of classifying a sample of material according to the modification of the embodiment of the present invention, principal component analysis may be performed on the set of arc-tangents $\theta_{31n}$ to obtain a principal component score comp.5.

8

Classification step

When the eighth analysis step, the fifth set of arc-tangents calculation step, and the fifth principal component analysis step are included, the principal component score comp.5 may be plotted on the third axis, the principal component score comp.1 may be plotted on the first axis, and the principal component score comp.3 may be plotted on the second axis. This enables comparison of pieces of time series data obtained by three different analysis methods. As a result, the sample of material can be classified more accurately.

The principal component score comp.4 may be plotted on the third axis, the principal component score comp. 1 may be plotted on the first axis, and the principal component score comp.2 may be plotted on the second axis. The principal component score comp.2 and the principal component score comp.3 are results obtained by the same analysis method and the principal component score comp.4 and the principal component score comp.5 are results obtained by the same analysis method. Further, the principal component score comp.3 represents information on the sample of material later in time than the principal component score comp.2 and the principal component score comp.5 represents information on the sample of material later in time than the principal component score comp.4. This enables comparison of the time dependence of a plurality of principal component scores. This makes it easier to extract time-dependent properties of the sample of material. As a result, the sample of material can be classified more accurately.

A third coordinate position represented by the principal component score comp.1, the principal component score comp.2, and the principal component score comp.4 and a fourth coordinate position represented by the principal component score comp.1, the principal component score comp.3, and the principal component score comp.5, which are plotted in the coordinate system, may be compared. This makes changes in the properties of the sample of material over time more obvious. As a result, the sample of material can be classified more accurately. A method of comparing the third coordinate position and the fourth coordinate position may use, for example, the direction or magnitude of displacement from the third coordinate position to the fourth coordinate position. This makes it possible to more quantitatively extract time-dependent properties of the sample of material. As a result, the sample of material can be classified more accurately.

Although methods of classifying a sample of material according to an embodiment and modifications of the present invention have been described above, the specific configurations thereof are not limited to those of the embodiment and modifications and configuration changes, configuration combinations, and configuration deletions can be made without departing from the spirit of the present invention. The configurations shown in the embodiment and modifications can also be appropriately combined.

The method of classifying a sample of material according to the present invention may measure a sample of material using one analysis method at four or more different times. This makes it easier to extract time-dependent properties of the sample of material.

In the method of classifying a sample of material according to the present invention, upon measurement of analysis values $I_1$ and $I_3$ at times $t_1$ and $t_3$, $I_2$ at $t_2$ satisfying $t_1 \leq tz \leq t_3$ may be calculated based on the analysis values $I_1$ and $I_3$. For example, $I_2$ may be calculated such that $I_2 = \{(I_3 - I_1)/(t_3 - t_1)\}(t_2 - t_1)$. This makes it easier to compare pieces of data obtained by different analysis methods.

The method of classifying a sample of material according to the present invention may be implemented by computer programming.

[Examples]

An example of the present invention will be specifically shown and described in more detail below, but the present invention is not limited to the following examples.

Samples of material

A plurality of paints with various differences such as oil and water-based paints and those with different colors or contained ingredients and coats of paint formed by them were used as samples of material. Paints have various differences, and when paint is applied, the properties of the paint change and finally become a coat of paint. The process of changing from paint to a coat of paint and the final coat of paint were analyzed by different analysis methods.

Commercially available paint Nos. 1 to 15 used for classification are shown below.

Oil-based #1, Maker: Rock Paint
No. 1 (maker's color: white), No. 2 (maker's color: super red), No. 3 (maker's color: deep blue), and No. 4 (maker's color: bright yellow white)
Oil-based #2, Maker: Nippon Paint
No. 5 (maker's color: white), No. 6 (maker's color: thread red), No. 7 (maker's color: vacation blue), and No. 8 (maker's color: extra yellow)
Water-based #1, Maker: Nippon Paint
No. 9 (maker's color: white), No. 10 (maker's color: red), No. 11 (maker's color: blue), and No. 12 (maker's color: yellow)
Water-based #2, Maker: Akzo Nobel
No. 13 (maker's color: white), No. 14 (maker's color: red), No. 15 (maker's color: blue), and No. 16 (maker's color: yellow)
Water-based #3, Maker: Asahipen
No. 17 (maker's color: red)
Water-based #4, Maker: Daiso
No. 18: Daiso (maker's color: red)

Analysis methods

A Fourier transform infrared spectrometer (FTIR) (Spectrum two, PerkinElmer Inc.) was used to measure the infrared spectrum (IR spectrum) of the paint or coat of paint. Gas chromatography-mass spectrometry (GC/MS) and a head space sampler (HS) (Clarus SQ8 GC/MS, PerkinElmer Inc.) were used to measure the volatile components of the paint or coat of paint. Inductively coupled plasma mass spectrometry (ICP-MS) (NexION2000, PerkinElmer Inc.) was used to analyze the inorganic elemental components of the paint or coat of paint. A thermogravimetric device (TG, TGA8000, PerkinElmer Inc.) was used for paint change rate analysis.

A Teflon (registered trademark) tape was pasted on a slide glass and paint was dripped on it. The paint was applied using a bar coater (with a wet film thickness of 100 μm) such that the thickness of the paint was constant. TG was used in the process of forming a coat of paint. Also, the sampling times were set at times when the drying time of the paint reached 1H, 6H, 24H, and 48H such that sampling could be performed at times exceeding the time when the mass loss of paint is stabilized. Then, the sampled paint was measured by various devices.

IR and ICP-MS measured coats of paint sampled at the drying times of 1H, 6H and 48H, and GC/MS measured coats of paint sampled at the drying times of 1H and 24H. In ICP-MS, the sampled coat of paint was acid-decomposed with nitric acid or a mixed acid of nitric acid and hydrofluoric acid (TAMAPURE AA-100, Tama Chemicals Co., Ltd.) by a microwave decomposition device, Titan MPS (PerkinElmer Inc.), before the measurement.

Normalization of analysis results

The paint analysis results differ in dimension and magnitude. Therefore, after main peaks were picked up from the analysis results obtained by each device, 23 peak intensities were extracted from the IR measurement results, the quantitative results of the concentrations of 61 elements were extracted from the ICP-MS measurement results, and 99 peak areas were extracted from the GC measurement results.

The analysis results were normalized by two methods. The first is a method of normalizing only the intensities of analysis results (a reference example). The second is a method of simultaneously normalizing the intensities and times of analysis results.

First, the method of normalizing only the intensities of the analysis results will be described. It can be assumed that the corrections between the analysis results of coats of paint obtained from the various analysis results are obtained from matrices representing the properties of samples. A characteristic data group composed of various analysis results is a result of extracting singular points and one singular point obtained by an analytical device can be regarded as corresponding to one property. Here, using an increment $x_n$ and a function F indicating an axis obtained by the analytical device, $x_1$ and $x_n+1$ can be expressed as follows:

$$x_1 = F(x_0) \ ... \ \text{Equation 1}$$

$$x_{n+1} = F(x_n) \ ... \ \text{Equation 2}$$

It can also be expressed as increment $\chi i = F(x_i, x_{i+i})$. n and i represent positive integers. If F is differentiable with respect to the characteristic axis, the relationship between the analytical device result $x_n$ and a data intensity $I_n$ at $x_n$ can be expressed as $I_n = G(F(x_n))$. Then, the entire data group composed of different intensities can be normalized as follows using the intensity I and the angle θ to take into account the intensity of movement from $x_i$ to $x_{i+1}$ that differs for each device.

$$\theta_n = \tan((I_n - I_{n-1}))^{-1} \dots \text{Equation 3}.$$

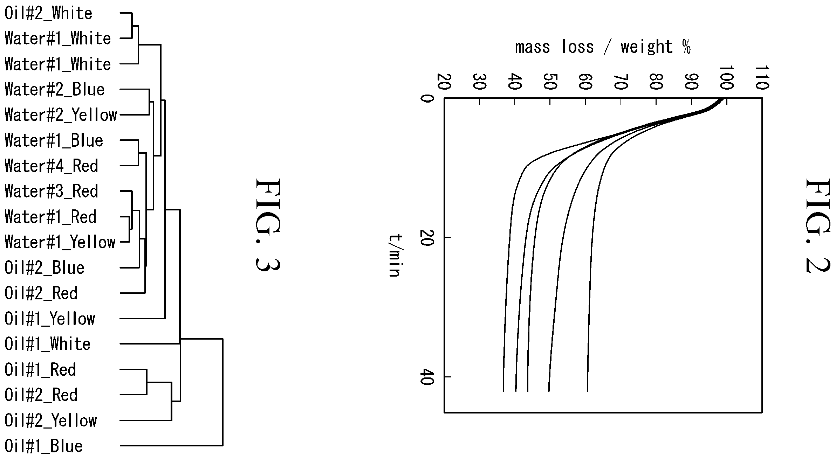

Next, the method of simultaneously normalizing the intensities and times of analysis results will be described. From the results of TG, the rate of mass loss due to evaporation of the solvent contained in the paint can be regarded as constant after 40 minutes as shown in FIG. 2. Measured values for samples of coats of paint at drying times at which the devices did not perform the measurement were calculated using a linear regression line. Thereby, intensity data with unified time was obtained for each device. Further, when intensities $I_1$ and $I_3$ are measured at times $t_1$ and $t_3$, an intensity $I_2$ at $t_2$ that satisfies $t_1 \leq t_2 \leq t_3$ can be expressed as $I_2 = \{(I_3 - I_1)/(t_3 - t_1)\}(t_2 - t_1)$, $I_n$ can be expressed as follows.

$$I_n = \{(I_3 - I_1)/(t_3 - t_1)\}(t_n - t_1) \dots \text{Equation 4}$$

$\theta_1$, which is the direction of displacement from (time $t_1$, intensity $I_1$) to (time $t_2$, intensity $I_2$) after time $\Delta t_1$ has elapsed, is given by the following equation.

$$\theta_1 = \tan((I_2 - I_1)/(t_2 - t_1))^{-1} \dots \text{Equation 5}$$

Similarly, $\theta_2$ after time $\Delta t_2$ has elapsed is given by the following equation.

$$\theta_2 = \arctan((I_3 - I_2)/(t_3 - t_2)) \dots \text{Equation 6}$$

Thus, In is normalized together with time $\Delta$tn as follows.

$$\theta_n = \arctan((I_{n+1} - I_n)/(t_{n+1} - t_n)) \dots \text{Equation 7}$$

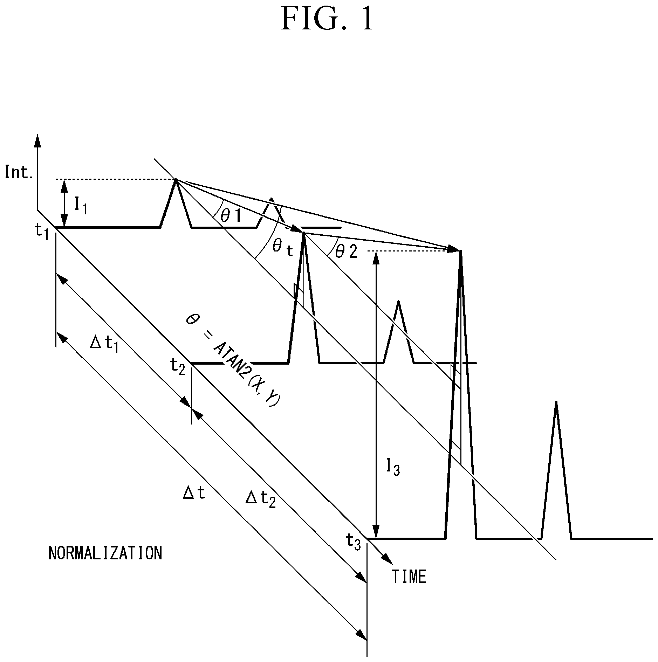

The normalization image is shown in FIG. 1.

PCA analysis

Analysis results obtained by each device can be regarded as being composed of singular points exhibiting nonlinearity. Therefore, if the $\theta$ data group obtained by normalization is regarded as a matrix model with an increment incorporating sample properties such that $\theta = \arctan(F(x), G(F(x)))$, it is possible to normalize the $\theta$ data group incorporating time change. As a result, analysis results can be converted into a matrix data group independent of intensities obtained from the devices.

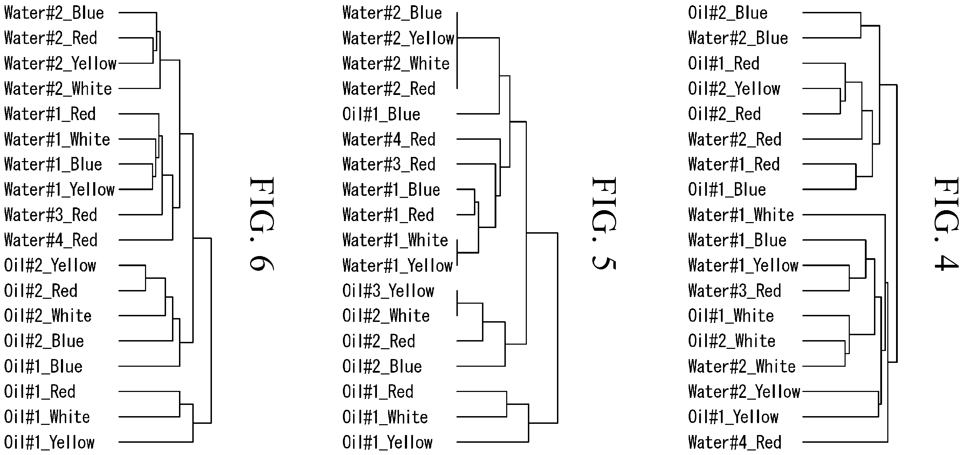

PCA analysis was performed on the normalized $\theta$ data group created by Equation 3 and PCA analysis was performed. FIGS. 3, 4 and 5 show the results of extracting analysis results of each device alone from a principal component score group and FIG. 6 shows the results of combining IR, ICP-MS, and GC/MS.

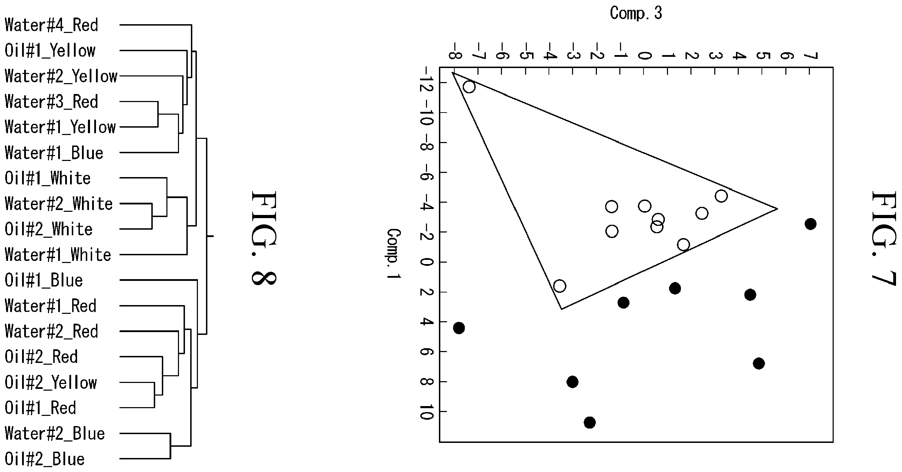

PCA analysis was performed on the normalized $\theta$ data group created by Equation 6 and $PCA_1$ score values comp. 1 to 3 at $\Delta t_1$ and $PCA_2$ score values Comp. 1' to 3' at $\Delta t_2$ which is time later than $\Delta t_1$ were calculated. A loadings score group was also calculated accompanying the principal component score group. FIG. 7 shows the results of combining IR, ICP-MS, and GC/MS extracted from these principal component score groups.

The principal component score group and the loadings score group of each sample are component values calculated from $\theta$ derived from the sample and can be regarded as unique features detected by the analytical device. Therefore, considering characteristic function axes that indicate unique features of the sample, analysis can be performed by integrating data with some of the PCA axes of the data group fixed and the other PCA axes changed (hereinafter sometimes referred to as PCAmerge). Therefore, Comp.2 and 3 and Comp.2' to 3' of $PCA_2$ with respect to Comp.1 of $PCA_1$ were plotted on three axes and changes in the center of gravity of each sample were investigated by PCAmerge. Changes in each measurement value $\theta$ reflecting changes in the center of gravity due to PCAmerge were also visualized by merging a loadings plot in the same manner.

Considerations

First, the case where only the intensities of analysis results were normalized was considered. When the normalized data group of IR, ICP-MS, or GC/MS was used alone as shown in FIGS. 3, 4 and 5, the dendrogram included a mixture of water-based paint and oil-based paint and the paints were not able to be separated appropriately. On the other hand, when the entire normalized data groups of IR, ICP-MS, and GC/MS are used as shown in FIG. 6, the paints were able to be separated into water-based and oil-based clusters. This means that the properties of samples of material can be easily and appropriately classified by normalizing pieces of data obtained by different analysis methods such that they can be compared with each other and using a plurality of analysis results in combination.

Further, when the results of PCA analysis of the data groups of the three analysis results were plotted, the distributions of water-based paint (hollow circles) and oil-based paint (solid circles) differ as shown in FIG. 7. From these results, it was found that even if separation is unclear in the analysis of results obtained with a single device, separation can be achieved by normalizing data from a plurality of analytical devices using Equation 3. It also means that the normalization of Equation 3, which handles a large amount of data of the devices without truncation, enabled conversion into comparable intensities without removing, from the data groups of paint, data groups indicating features derived from materials such as solvents and solutes included therein.

FIG. 8 shows a dendrogram in which two device data groups of IR and ICP-MS are extracted from the normalized data groups. Paint colors (red, white, blue, and yellow) were not clearly separated when the two device data groups of IR and ICP-MS were used. This suggests a lack of color information in the analysis data groups of IR and ICP-MS. Thus, using Equation 3 that normalizes data groups of multiple devices, the analysis results were expressed as clusters if they contain data indicating target features and expressed as variances if they do not contain target features. Using the dendrogram of FIG. 8, it was possible to determine the lack of information on required features in the data groups. As a result, it was possible to determine whether the types and results of analytical devices used for characteristic analysis were appropriate.

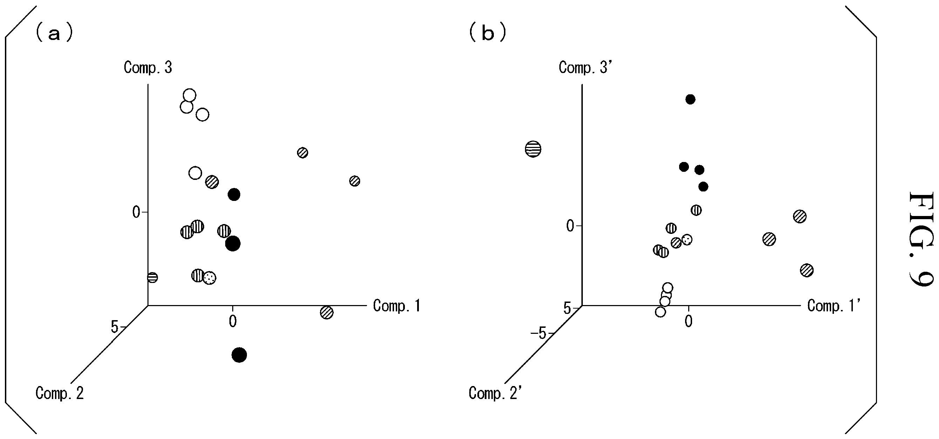

Next, the case where the intensities and times of analysis results were normalized simultaneously was considered. The results of TG showed that the rate of mass loss was constant when 40 minutes or more elapsed from application and thus it was determined that the changes of samples could be linearly approximated. Further, in order to accurately approximate an intensity change function, 1H to 6H after application was defined as an elapsed time $\Delta t_1$ and 6H to 24H after application was defined as an elapsed time $\Delta t_2$ and the intensities and times were normalized after linear approximation of the intensity change rate in each range, the normalized plots of which are shown in FIG. 9.

Looking at a graph from 1H to 6H after application (FIG. 9 left) and a graph from 6H to 24H after application (FIG. 9 right), clusters for each maker were confirmed. On the other hand, it is considered that the difference in cluster distribution between 1H to 6H after application and 6H to 24H after application is due to the fact that principal component analysis was performed individually for each of data groups formed at times at which the analysis of orthogonal axes in principal component analysis differs, despite identical samples. That is, it is considered to mean that the characteristic axes of the same sample change. When it was possible to compare the progresses of various reactions such as deterioration and hardening with the same sample in the device analysis of the process of changing the states of materials, it was possible to determine that the clusters of the results in FIG. 9 were expressed as results indicating the properties of the sample. On the other hand, when the states of a sample change with the progress of desolvation like as paint does, various analysis results indicate transient states of the sample and it is difficult to compare the progresses of various reactions with the same sample. That is, it was possible to determine that the cluster analysis using device analysis in the transient state analysis using analytical devices was not sufficient to classify paints.

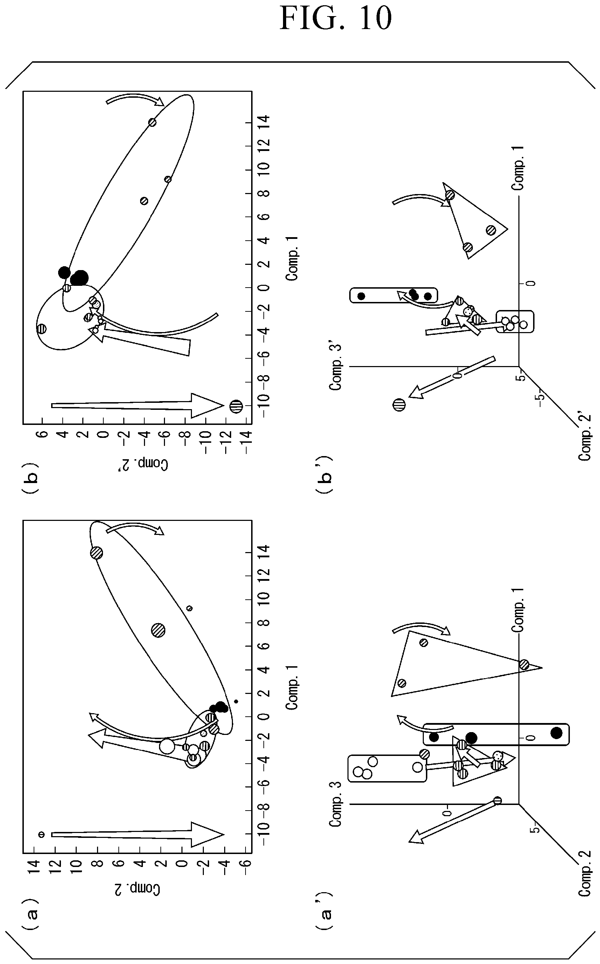

Therefore, time was also incorporated to perform analysis. When it is assumed that solute dissolution, gas diffusion, and chemical reactions are based on classical mechanics in which something moves while maintaining linearity, there is a need to consider chemical reactions in the form of A + B to C + D using a non-equilibrium model of avrami et al., involving reaction rates. Thus, assuming that FIG. 9 shows the analysis results of equilibrium, the times of analysis results were matched using Equations 4 and 7 and the data intensities were normalized to create normalized data groups with unified dimensions. For these normalized data groups including time, Comp.2' and Comp.3' of PCA2 were calculated with Comp.1 of FIG. 9 fixed as a reference axis, the plotted results of which are shown in FIG. 10. Graphs a and a' of FIG. 10 show the same results. Graphs b and b' show the same results.

Attention was paid to the movement of the score plot as time changes from time $\Delta t_1$ (graph a of FIG. 10) to time $\Delta t_2$ (graph b of FIG. 10). Water-based #1 moved negatively in the directions of comp.2 and comp.3. Water-based #2 and #3 moved positively in the direction of comp.2. Water-based #4 moved negatively in the direction of comp.2 and positively in the direction of comp.3. Oil-based #1 moved rotationally. Oil-based #2 moved positively in the directions of comp.2 and comp.3. It was confirmed that movements of the centers of gravity of features were different for each paint as described above. Through PCA analysis of such normalized data groups including time, it was confirmed that products of the same maker formed clusters while causing score movements in the same direction.

Regarding such anisotropy of movements, the classification of differences between makers was unclear according to the results in FIGS. 6 to 8. On the other hand, by performing PCA with the reference axis set using the data groups of properties with reference to time shown in FIG. 10, it was possible to obtain the relationship between the principal component scores. This made it possible to obtain the correlation of analysis data. In addition, using the analysis method considering time, it was possible to classify differences of paints including differences between makers. For the same samples, the normalized θ data groups of the present method exhibit Gaussian distributions with concentric circles and therefore it is considered that they do not indicate different changes in the centers of gravity unless the movements of the centers of gravity are different. It is considered that differences in the centers of gravity of samples were appropriately expressed in FIG. 10 as features of the samples. Thus, the method used in the present example was able to appropriately classify samples.

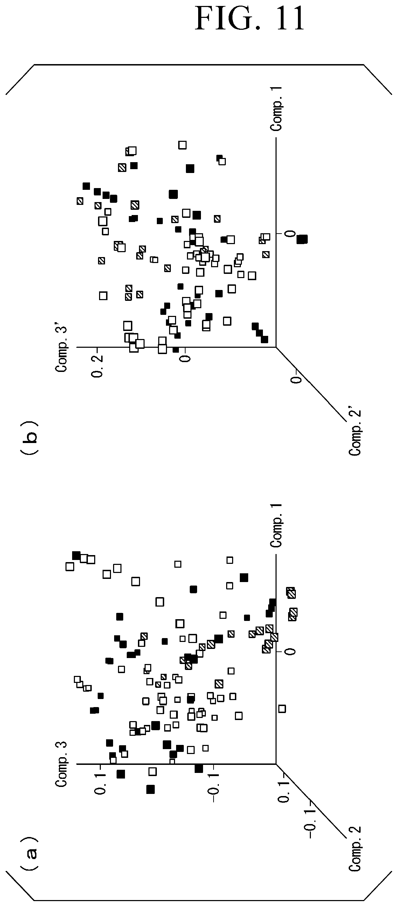

When the properties of paints are shown in PCA loadings plots (in FIG. 11) of the data of various analytical devices that make up the results of FIG. 10, ICP-MS (hollow) plots show a shift to a plane from a variance and IR plots show a variance from a plane. From the results of FIG. 10, it was possible to determine that the plotted positions of IR measurement components tend to change greatly with respect to the whole. The results of FIG. 11 can be regarded as factors in the shifts of FIG. 10 because the movements of scores indicate changes in information indicating features of samples. Thus, it was possible to investigate the causative elements of the movements of clusters of features obtained from the results of FIG. 10 using the PCA loadings plots. Accordingly, from the results of FIG. 10, it was possible to extract changes in the properties of samples and the validities of the analysis methods. From the results of FIG. 10, it was also possible to extract important techniques for analyzing the properties of samples.

[Industrial Applicability]

The present invention can provide a method of classifying a sample of material that enables comparison of pieces of time series data obtained by different analysis methods and thus has high industrial applicability.

**Claims**

1. A method of classifying a sample of material, the method comprising:

    a first analysis step of analyzing a sample of material using a first analysis method at a time $t_1$ to obtain a first set of analysis values ($I_{111}$, $I_{112}$...$I_{11n}$);
    a second analysis step of analyzing the sample of material using the first analysis method at a time t2 which is later than a time t1 to obtain a second set of analysis values ($I_{121}$, $I_{122}$, ..., $I_{12n}$);
    a first set of arc-tangents calculating step of obtaining a set of set of arc-tangents ($\theta_{1n}$) of respective ratios of analysis value differences ($I_{12n}$ - $I_{11n}$) to a time difference (t2 - 11) between the time (t2) and the time (t1);
    a first principal component analysis step of performing a principal component analysis of the set of set of arc-tangents ($\theta_{1n}$) to obtain a principal component score (comp.1);
    a third analysis step of analyzing the sample of material using a second analysis method different from the first analysis method at a time (t3) to obtain a third set of analysis values ($I_{231}$, $I_{232}$, ..., $I_{23n}$);
    a fourth analysis step of analyzing the sample of material using the second analysis method at a time (t4) which is later than the time (t3) to obtain a fourth set of analysis values ($I_{241}$, $I_{242}$, ..., $I_{24n}$);
    a second set of arc-tangents calculation step of obtaining a set of set of arc-tangents ($\theta_{2n}$) of respective ratios of analysis value differences ($I_{24n}$ - $I_{23n}$) to a time difference (t4 - t3) between the time (t4) and the time (t3);
    a second principal component analysis step of performing a principal component analysis of the set of set of arc-tangents ($\theta_{2n}$) to obtain a principal component score (comp.2); and
    a classification step of plotting the principal component score (comp.1) on a first axis and the principal component score (comp.2) on a second axis of a coordinate system having the first and second axes and of classifying the sample of material based on the principal component scores (comp.2) of the second analysis method with respect to the principal component scores (comp.1) of the first analysis method.

2. The method according to claim 1, further comprising:

    a fifth analysis step of analyzing the sample of material using the second analysis method at a time (t5) which is later than the time (t4) to obtain a fifth set of analysis values ($I_{251}$, $I_{252}$, ..., $I_{25n}$);
    a third set of arc-tangents calculation step of obtaining a set of set of arc-tangents ($\theta_{21n}$) of respective ratios of value differences ($I_{25n}$ - $I_{24n}$) to a time difference (t5 - t4) between the time (t5) and the time (t4); and
    a third principal component analysis step of performing a principal component analysis of the set of set of arc-tangents ($\theta_{21n}$) to obtain a principal component score (comp.3),
    wherein the classification step further includes plotting the principal component score (comp. 1) on the first axis and the principal component score (comp.3) on the second axis in the coordinate system having the first and second axes to classify the sample of material.

3. The method according to claim 2, wherein the classification step further includes comparing a first coordinate position and a second coordinate position,

wherein the first coordinate position is defined by the principal component score (comp. 1) and by the principal component score (comp.2) which are plotted on the coordinate system, and
wherein the second coordinate position is defined by the principal component score (comp. 1) and the principal component score (comp.3) which are plotted in the coordinate system.

4. The method according to claim 3, wherein the classification step further includes comparing the first coordinate position and the second coordinate position based on a direction of displacement defined from the first coordinate position to the second coordinate position.

5. The method according to claim 2, further comprising:

a sixth analysis step of analyzing the sample of material using a third analysis method different from the first and second analysis methods at a time (t6) to obtain a sixth set of analysis values ($I_{361}$, $I_{362}$, ..., $I_{36n}$);
a seventh analysis step of analyzing the sample of material using the third analysis method at a time (t7) which is later than the time (t6) to obtain a seventh set of analysis values ($I_{371}$, $I_{372}$, ..., $I_{37n}$);
a fourth set of arc-tangents calculation step of obtaining a set of set of arc-tangents ($\theta_{3n}$) of respective ratios of analysis value differences ($I_{37n}$ - $I_{36n}$) to a time difference (t7 - t6) between the time (t7) and the time (t6); and
a fourth principal component analysis step of performing a principal component analysis of the set of set of arc-tangents ($\theta_{3n}$) to obtain a principal component score (comp. 4),
wherein the classification step further includes plotting the principal component score (comp.4) on a third axis of the coordinate system, the principal component score (comp. 1) on the first axis, and the principal component score (comp.2) on the second axis in the coordinate system having the first, second, and third axes.

6. The method according to claim 5, further comprising:

an eighth analysis step of analyzing the sample of material using the third analysis method at a time (t8) which is later than the time (t7) to obtain an eighth set of analysis values ($I_{381}$, $I_{382}$, ..., $I_{38n}$);
a fifth set of arc-tangents calculation step of obtaining a set of set of arc-tangents ($\theta_{31n}$) of respective ratios of a set of analysis value differences ($I_{38n}$ - $I_{37n}$) to a time difference (t8 - t7) between the time (t8) and the time (t7); and
a fifth principal component analysis step of performing a principal component analysis of the set of set of arc-tangents ($\theta_{31n}$) to obtain a principal component score (comp. 5),
wherein the classification step includes plotting the principal component score (comp.5) on the third axis, the principal component score (comp.1) on the first axis, and the principal component score (comp.3) on the second axis in the coordinate system having the first, second, and third axes.

7. The method according to claim 6, wherein the classification step further includes comparing a third coordinate position and a fourth coordinate position,

wherein the third coordinate position is defined by the principal component score (comp.1), the principal component score (comp.2), and the principal component score (comp.4) which are plotted in the coordinate system, and
wherein the fourth coordinate position is defined by the principal component score (comp.1), the principal component score (comp.3), and the principal component score (comp.5) which are plotted in the coordinate system.

8. The method according to claim 7, wherein the classification step further includes comparing the third coordinate position and the fourth coordinate position based on a direction of displacement defined from the third coordinate position to the fourth coordinate position.

9. The method according to claim 1, wherein the first and second analysis methods are different from each other in terms of types of physical quantities for analysis.

# FIG. 1

# FIG. 2

# FIG. 3

# FIG. 4

# FIG. 5

# FIG. 6

# FIG. 7

# FIG. 8

# FIG. 9

# FIG. 10

FIG. 11

## INTERNATIONAL SEARCH REPORT

| | International application No. |
|---|---|
| | PCT/JP2020/041928 |

| A. CLASSIFICATION OF SUBJECT MATTER |
|---|
| G01N 33/00(2006.01)i |
| FI: G01N33/00 D |
| According to International Patent Classification (IPC) or to both national classification and IPC |

| B. FIELDS SEARCHED |
|---|
| Minimum documentation searched (classification system followed by classification symbols) |
| G01N33/00 |

Documentation searched other than minimum documentation to the extent that such documents are included in the fields searched
| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2016-195974 A (SUMITOMO CHEMICAL CO., LTD.) 24 November 2016 (2016-11-24) paragraphs [0032]-[0072] | 1-9 |
| A | JP 2011-223957 A (OSAKA UNIVERSITY) 10 November 2011 (2011-11-10) paragraphs [0096]-[0100] | 1-9 |
| A | JP 05-297888 A (OKI ELECTRIC INDUSTRY CO., LTD.) 12 November 1993 (1993-11-12) paragraphs [0010]-[0021] | 1-9 |
| A | JP 2020-030089 A (HITACHI HIGH-TECHNOLOGIES CORP.) 27 February 2020 (2020-02-27) paragraphs [0104]-[0111] | 1-9 |
| A | JP 2010-230428 A (TOHOKU UNIVERSITY) 14 October 2010 (2010-10-14) paragraphs [0022]-[0046] | 1-9 |

☐ Further documents are listed in the continuation of Box C.  ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 22 January 2021 (22.01.2021) | 02 February 2021 (02.02.2021) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

22

**INTERNATIONAL SEARCH REPORT**
Information on patent family members

| International application No. |
| --- |
| PCT/JP2020/041928 |

| Patent Documents referred in the Report | Publication Date | Patent Family | Publication Date |
| --- | --- | --- | --- |
| JP 2016-195974 A | 24 Nov. 2016 | US 2018/0105858 A1<br>paragraphs [0035]-[0075]<br>WO 2016/159154 A1<br>CN 107531528 A<br>KR 10-2017-0132329 A | |
| JP 2011-223957 A | 10 Nov. 2011 | US 2011/0263444 A1<br>paragraphs [0096]-[0100] | |
| JP 05-297888 A | 12 Nov. 1993 | (Family: none) | |
| JP 2020-030089 A | 27 Feb. 2020 | CN 110857946 A<br>paragraphs [0251]-[0268]<br>DE 102019212625 A1 | |
| JP 2010-230428 A | 14 Oct. 2010 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008102825 A
- JP H5324272 A
- JP 2018535471 W
- JP 2008077677 A